# EUROPEAN PATENT SPECIFICATION

(11) **EP 1 890 628 B1**
(45) Date of publication and mention of the grant of the patent: **01.01.2014**
(21) Application number: 06733337.7
(22) Date of filing: 26.04.2006
(51) Int. Cl.: A61B 19/00

(54) **CONNECTING DEVICE FOR CONNECTING A RING-SHAPED FRAME TO A SUPPLEMENTARY EQUIPMENT**
VERBINDUNGSVORRICHTUNG ZUR VERBINDUNG EINES RINGFÖRMIGEN RAHMENS MIT EINEM HILFSGERÄT
DISPOSITIF DE CONNEXION D'UN CADRE ANNULAIRE A UN INSTRUMENT

(30) Priority: 29.04.2005 SE 0501002
(43) Date of publication of application: 27.02.2008
(73) Proprietor: Elekta AB (PUBL), 103 93 Stockholm (SE)
(72) Inventor: ARN, Thomas, S-181 46 Lidingö (SE)
(74) Representative: Skagersten, Thomas
(86) International application number: PCT/SE2006/000481
(87) International publication number: WO 2006/118509

(56) References cited:
- EP-A1- 0 522 242
- US-A- 4 475 550
- US-A- 5 040 547
- US-A- 5 040 547
- US-A- 5 417 672
- US-A- 5 423 832
- US-A- 5 423 832
- US-A- 5 855 582
- US-A1- 2004 123 870

## Description

The invention relates to a connecting device for connecting a ring-shaped frame, which is adapted for fixation to the head of a patient during neurological diagnosis, therapy or surgery, to a supplementary equipment, for positioning of the frame and the equipment in a predetermined relation to each other, wherein the frame comprises a connecting bore or hole and the equipment comprises a connecting pin for insertion into the hole in the frame during positioning.

### Background of the invention

During neurological diagnosis, therapy or surgery of the head of a patient, use is made of a ring-shaped frame, which is secured to the head by means of at least three pointed fixation pins which are tightened against the skull for fixation of the frame thereto.

However, when tightening of the fixation pins against the skull, which usually is accomplished by threaded fixation pins which are screwed into the skull, the frame will be deformed due to the large forces acting between the frame and the skull by means of the fixation pins. The deformation will be particular significant when, as is common practice today, using pin support members, which project upwards from the frame, with the fixation pins passed through the upper ends of each pin support member. Hereby the moment forces acting on the frame, will be considerable.

The deformation of the frame makes it more difficult to connect supplementary equipment, needed for the diagnosis, therapy or surgery, to the frame in a proper position. When performing diagnosis, therapy or surgery proceedings to the head, it is for obvious reasons extremely important that the area to be treated, is localized with a large precision, as well as that the treatment is performed in the proper area, which necessitates positioning of the equipment with large precision on the frame.

More precisely, when diagnosing and locating areas in the head to be treated, it is common practice to perform some kind of imaging of the head, e.g. by means of Magnetic Resonance Imaging, and for this purpose it is known in the art to place an indication box, having fiducials or reference markings, which are visible in the imaging method used, on the frame to be able to establish the frame position in the images. It is therefore important to be able to position the frame and the indication box in an as accurate defined relationship as possible. For this purpose it is known in the art to provide the frame with guiding and connecting holes or bores and the indication boxes with matching pins to locate the indication box correct on the frame. At least two bores and pins, respectively, are required for proper positioning. To enable firm holding of the indicating box onto the frame, there are generally also provided some other kind of fixation device such as one or more tensioning clamps.

However, as the frame is deformed during attachment on the head, a certain play must be provided between the holes in the frame and the pins on the indication box, in order to allow mounting and removal of the indicating box while the frame is attached to the head. Consequently, the frame and indication box are somewhat movable in relation to each other, which decreases the precision of the identified position during imaging.

Other types of supplementary equipment which might be desired to mount on a frame of this kind, is e.g. a graduated scale and/or a guide member for positioning and displaceable mounting of instruments for diagnosis, treatment or surgery, for example surgical instruments or needles for taking of samples or treatment. Furthermore, the frame is often used as a fixture for steady mounting of the patient's head to different kinds of equipments or apparatuses, such as image producing equipment, for image scanning of the head, or equipment for radiation treatment of chosen areas inside the head. The same problems as in connection with the indicating boxes above, applies also here, i.e. due to the deformations of the frame when attaching it to the head, it is necessary to provide a play between the holes and the pin in, order to allow mounting and removal of the equipment, with decreased accuracy, of the equipment position as a result.

The preamble of claim 1 is based on US-A-5040547.

### Summary of the invention

The object of the invention is to eliminate or reduce the above mentioned problems associated with prior art devices, and provide a connecting device for interconnecting a ring-shaped frame and a supplementary equipment with an enhanced precision. At least this object is achieved by a device according to claim 1.

Accordingly, the object is achieved by a device comprising connecting holes formed in the frame and connecting pins on the equipment, wherein each hole opens into a surface of the frame and has an enlarged portion, which preferably is outward diverging, closest to the surface and a cylindrical portion beyond the enlarged portion and comprising the additional features of claim 1.

The reason why such a design of the holes and pins is advantageous in respect of the precision when interconnecting the frame and the equipment to each other, is that when deforming of an object, which in the case of a frame can be approximated to a beam, by applying a bending moment to it, one side of the object will be exposed to tensile stresses whereas the other side will be exposed to compressive stresses. Accordingly, due to the deformation, one side will be displaced in one direction and the other side will be displaced in the other direction. Somewhere there between, usually in the middle, the tensile forces, and the displacement, will accordingly be zero. This position is called the neutral layer. By locating the cylindrical portion of each hole in or near the neutral layer and make it appropriate short, this part of the hole will not, or at least to a small extent, be displaced and, consequently, no or only a small play has to be provided between the holes and the pins in order to compensate for the deformation and resulting displacement. By designing the holes with an enlarged portion closest to the opening in the surface of the frame, this portion of the frame will not affect the pin with a misalignment or bending moment, despite any deformation, and the equipment will be easy to mount and remove even when the frame is attached to the head.

Although the holes are designed as outlined above, in a preferred embodiment of the invention, the holes and the pins are located in positions where the deformation of the frame, when attached to a head, will be small or minimal. Such positions may be calculated by any calculation method known in the art, such as by the finite element method (FEM-analysis), or be measured upon loading of the frame.

Within the scope of the invention, it is envisaged that the pins may be cylindrical or have any other appropriate shape. However, according to the invention, the pins are formed with a bulge or a ball having a cross sectional dimension which is equal to or slightly smaller than the cross sectional dimension of the hole, whereas a shaft portion of the pins has a smaller cross sectional dimension. By this, the contact surface between the hole and the pin will be advantageously small so that the risk that a possible difference in angle between the hole and the pin will cause a bending moment on the pin, is further reduced.

In a hereinafter described and in the drawings illustrated embodiment of the invention, the holes and the pins are directed perpendicular to a plane of the ring-shaped frame, i.e. the holes are formed in the upper and lower edge of the frame. However, the invention is not limited to that embodiment. On the contrary, the holes and pins may be arbitrary directed, according to desire and requirement, e.g. also in a direction in parallel to the plane of the frame with the holes opening through the lateral surfaces of the frame. Provisions may even be made to attach equipment from two opposite directions, e.g. from above and below, as is shown in an embodiment in the drawings. Besides such an embodiment, it is however not necessary for the holes to extend completely through the frame. On the contrary, the holes can also be terminated inside the frame.

Two holes and pins are normally required to position and prevent rotation of an arbitrary equipment with large precision in a predetermined location. In order to enhance the precision further it is advantageous to design the holes and pins such that the holes are either circular in cross section and the pins are oval, or the holes are oval in cross section whereas the pins are circular. In this way the hole and pin pair will contribute to the exact positioning in one direction only. One such hole and pin pair may preferably be combined with an ordinary hole and pin pair which restrain the movement in all directions. Hereby, the risk of stresses between the equipment and the frame, due to deformation of the latter, is further reduced.

The enlarged portion of each hole may have any arbitrary, appropriate form. In a preferred embodiment it is conical and diverging towards the frame surface, but it is to be understood that it need not be linearly diverging in a longitudinal section. On the contrary it can also have the form of a cylindrical hole with larger diameter in relation to the pin guiding cylindrical portion further inward in the frame.

### Brief description of the drawings

The invention will hereinafter be described, by way of example, with reference to the accompanying drawings, in which:
- Fig 1: is a partly exploded perspective view of a frame, which is attached to the head of a patient, and an indication box which is to be mounted onto the frame;
- Fig 2-5: are longitudinal sectional views of different hole and pin embodiments; and
- Fig 6: is a cross sectional view through an embodiment of a hole and pin design.

### Description of preferred embodiments

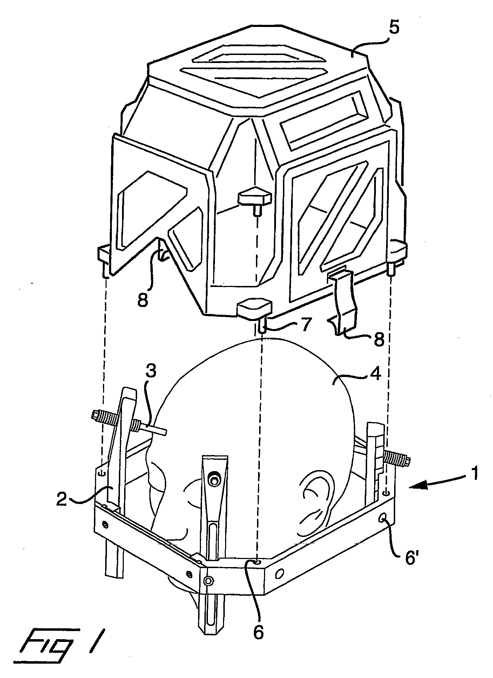

Reference is first made to fig 1 in which the field of application for the present invention, is illustrated in a perspective view. With reference number 1 is indicated a frame, which has a general rectangular shape and includes in each "corner" an upright pin support member 2, which each in an upper end holds fixation pins 3. Each fixation pin 3 has a pointed end which is bearable against the head 4 of a patient by threading through a hole in the pin support member 2 for rigid attachment of the frame towards the head.

When using the frame for imaging and diagnosing of areas inside the head, an indication box 5, having reference markings which are visible in the imaging method being used, can be mounted onto the frame as is indicated in fig 1. For this purpose, the frame is provided with holes 6 on the upper edge of the frame, whereas the indication box is provided with pins 7 on its bottom edge. When placing the indication box 5 onto the frame 1, such that the pins 7 are fitted into the holes 6, the indication box will be held in a correct defined position and is prevented from displacement in a horizontal direction, as viewed in fig 1, in relation to the frame. To secure the indication box to the frame and prevent displacement also in the vertical direction, the indication box is provided with two tensioning clamps 8 which engage the frame by gripping around its lower edge. In fig 1 is also shown holes 6' in the lateral surface of the frame, which extend in a direction parallel to the plane of the frame. These holes are adapted for alternative mounting of other types of equipment.

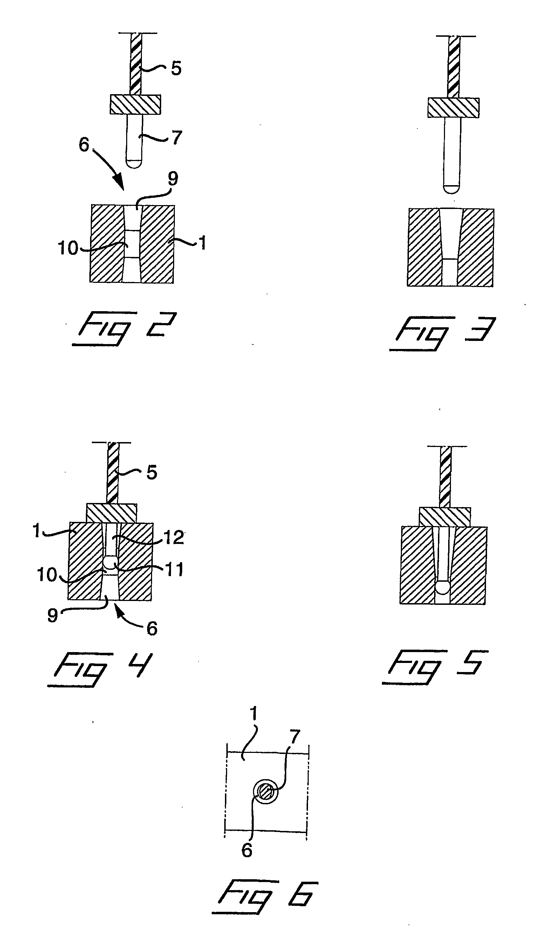

In figs 2-5, alternative designs of the hole 6 and pin 7 arrangement are shown in cross sections through the frame 1 and the lower portion of the indication box 5. In each of figs 2 and 3, the frame 1 and the indication box are separated from each other for better visibility. In these embodiments the pins are cylindrical with uniform cross sectional dimension over their entire lengths. According to the invention, the holes 6 in the frame, are composed of at least one enlarged portion in form of an outward diverging portion 9 adjacent a surface of the frame and a cylindrical portion 10 beyond the diverging portion. The example according to fig 2, includes a diverging portion 9 from two opposed surfaces and has an intermediate cylindrical portion 10 there between. In this way it is possible to attach equipment to the frame, in accordance with the invention, from above as well as from below. The length of the pin 7 is also reduced in fig 2 in comparison to fig 3.

By adaptation of the cross sectional dimension of the pin 7 such that it is equal to or slightly smaller than the cross sectional dimension of the cylindrical portion 10 of the hole 6, it is possible to obtain a well defined position for the pin 7 in the hole 6, and consequently a well defined position of the indication box 5 on the frame 1. At the same time, the diverging portion allows for easy mounting and removal of the indication box, despite any deformation of the frame, since the deformation will affect the surface areas of the frame mostly, whereas the cylindrical portion, which is located in or in the vicinity of the neutral layer, as has been described hereinbefore, will be comparatively unaffected.

In figs 4 and 5, is shown two alternative embodiments of the pin according to the invention, in two cross sectional views through the frame 1 and the lower portion of the indication box 5 with the pins inserted into the holes. The pins are shown in two different lengths. One short in fig 4 for a hole adapted for insertion from opposite directions, and one long pin in fig 5 for a hole adapted for insertion from one direction only. In these embodiments the pin is not of uniform thickness, as in the previous embodiments, but has a bulge or ball-shaped end 11, having a cross sectional dimension that is equal to or slightly smaller than the cross sectional dimension of the cylindrical portion 10 in the hole. The ball-shaped end of the pin is carried by a shaft portion 12 having a smaller cross sectional dimension than the end. With pins formed in this way, the contact surface between the pin and the hole will be advantageously small having to effect that larger angle differences between the pin and the hole, due to deformation, is possible.

In fig 6 is shown a further alternative embodiment of the pin and hole arrangement according to the invention, in a view/cross section seen from above in the direction of the pin and hole. The pin 7 may either be of the type having uniform thickness, as in figs 2 and 3, not according to the invention, or of the type having a ball-shaped end as in figs 4 and 5. As can be seen from the figure, the hole 6 in the frame 1 has a circular shape in cross section, whereas the pin 7 has an oval shape in cross section. In this way a two-point contact is obtained between the pin and the hole, such that exact guiding is obtained only in two opposite directions. By combining different pin and hole arrangements guiding in different directions, certain advantages may be achieved at deformation of the frame, as has been described earlier. Instead of a circular hole and an oval pin, the same effect can be achieved by an oval hole and a circular pin.

## Claims

1. Connecting device for connecting a ring-shaped frame (1), which is adapted for fixation to the head (4) of a patient during neurological diagnosis, therapy or surgery, to a supplementary equipment (5), for positioning of the frame and the equipment in a predetermined relation to each other, wherein the frame comprises a connecting hole (6) and the equipment comprises a connecting pin (7) for insertion into the hole in the frame during positioning, **characterized in that** the hole (6) opens into a surface of the frame (1) and has an enlarged portion (9) closest to the surface and a cylindrical portion (10), having a smaller cross sectional dimension than the enlarged portion, beyond the enlarged portion, whereas the connecting pin (7) has a ball-shaped end (11) which has a cross sectional dimension which is equal to or slightly smaller than the cross sectional dimension of the cylindrical portion (10) in the hole (6).

2. Connecting device according to claim 1, **characterized in that** the cylindrical portion (10) of the hole (6) is located at the neutral layer of the frame (1).

3. Connecting device according to any of the preceding claims, **characterized in that** the the hole (6) extends through the frame (1) and has an enlarged portion (9) at opposite surfaces of the frame and a cylindrical portion (10) between the enlarged portions.

4. Connecting device according to any of the preceding claims, **characterized in that** the holes (6) in the frame (1) are located in positions where the deformation of the frame is minimal when the frame is attached to a head.

5. Connecting device according to any of the preceding claims, **characterized in that** either the pin (7) or the hole (6) has an oval form in cross section, whereas the other is circular in cross section.

6. Connecting device according to any of the preceding claims, **characterized in that** the enlarged portion (9) is diverging towards the surface of the frame (1).

## Patentansprüche

1. Verbindungsvorrichtung zum Verbinden eines ringförmigen Rahmens (1), die geeignet ist zur Befestigung am Kopf (4) eines Patienten während einer neurologischen Diagnose, Therapie oder Operation an einer zusätzlichen Apparatur (5), um den Rahmen und die Apparatur in einer vorbestimmten Beziehung zueinander zu positionieren, wobei der Rahmen ein Verbindungsloch (6) umfasst und die Apparatur einen Verbindungsstift (7) zum Einfügen in das Loch in dem Rahmen während des Positionierens umfasst, **dadurch gekennzeichnet, dass** das Loch (6) in eine Oberfläche des Rahmens (1) mündet und einen vergrößerten Abschnitt (9) ganz in der Nähe der Oberfläche und einen zylindrischen Abschnitt (10), der eine kleinere Querschnittsdimension als der vergrößerte Abschnitt aufweist, hinter dem vergrößerten Abschnitt aufweist, wohingegen der Verbindungsstift (7) ein kugelförmiges Ende (11) aufweist, das eine Querschnittsdimension aufweist, die gleich oder geringfügig kleiner als die Querschnittsdimension des zylindrischen Abschnitts (10) in dem Loch (6) ist.

2. Verbindungsvorrichtung nach Anspruch 1, **dadurch gekennzeichnet, dass** sich der zylindrische Abschnitt (10) des Lochs (6) auf der neutralen Schicht des Rahmens (1) befindet.

3. Verbindungsvorrichtung nach einem der vorhergehenden Ansprüche, **dadurch gekennzeichnet, dass** sich das Loch (6) durch den Rahmen (1) hindurch erstreckt und einen vergrößerten Abschnitt (9) auf gegenüberliegenden Oberflächen des Rahmens und einen zylindrischen Abschnitt (10) zwischen den vergrößerten Abschnitten aufweist.

4. Verbindungsvorrichtung nach einem der vorhergehenden Ansprüche, **dadurch gekennzeichnet, dass** sich die Löcher (6) in dem Rahmen (1) in Positionen befinden, in denen die Verformung des Rahmens minimal ist, wenn der Rahmen an einem Kopf angebracht ist.

5. Verbindungsvorrichtung nach einem der vorhergehenden Ansprüche, **dadurch gekennzeichnet, dass** entweder der Stift (7) oder das Loch (6) eine im Querschnitt ovale Form aufweist, wohingegen der andere im Querschnitt kreisförmig ist.

6. Verbindungsvorrichtung nach einem der vorhergehenden Ansprüche, **dadurch gekennzeichnet, dass** der vergrößerte Abschnitt (9) in Richtung auf die Oberfläche des Rahmens (1) abweicht.

## Revendications

1. Dispositif de raccord pour le raccord d'un châssis (1) en forme d'anneau, adapté pour être fixé à la tête (4) d'un patient au cours d'un diagnostic neurologique, une thérapie ou une chirurgie, à un appareil supplémentaire (5), pour positionner le châssis et l'appareil de manière prédéterminée l'un par rapport à l'autre, dans lequel le châssis comprend un trou de raccord (6) et l'appareil comprend une broche de raccord (7) à insérer dans le trou du châssis pendant le positionnement, **caractérisé en ce que** le trou (6) débouche sur une surface du châssis (1) et possède une partie élargie (9) la plus proche de la surface et une partie cylindrique (10), ayant une dimension de coupe transversale inférieure à la partie élargie, au-delà de la partie élargie, alors que la broche de raccord (7) a une extrémité en forme de bille (11) qui a une dimension de coupe transversale égale ou légèrement inférieure à la dimension de coupe transversale de la partie cylindrique (10) dans le trou (6).

2. Dispositif de raccord selon la revendication 1, **caractérisé en ce que** la partie cylindrique (10) du trou (6) est située sur la couche neutre du châssis (1).

3. Dispositif de raccord selon l'une quelconque des revendications précédentes, **caractérisé en ce que** le trou (6) s'étend à travers le châssis (1) et présente une partie élargie (9) au niveau des surfaces opposées du châssis et une partie cylindrique (10) entre les parties élargies.

4. Dispositif de raccord selon l'une quelconque des revendications précédentes, **caractérisé en ce que** les trous (6) dans le châssis (1) sont situés à des positions où la déformation du châssis est minimale lorsque le châssis est fixé à une tête.

5. Dispositif de raccord selon l'une quelconque des revendications précédentes, **caractérisé en ce qu'**un élément entre la broche (7) ou le trou (6) possède une forme ovale en coupe transversale, tandis que le second élément est circulaire en coupe transversale.

6. Dispositif de raccord selon l'une quelconque des revendications précédentes, **caractérisé en ce que** la partie élargie (9) diverge vers la surface du châssis (1).
